# EUROPEAN PATENT APPLICATION

(11) **EP 3 998 024 A1**
(43) Date of publication of application: **18.05.2022**
(21) Application number: 19937148.5
(22) Date of filing: 09.07.2019
(51) Int. Cl.: A61B 17/02

(54) **DETACHABLE MULTI-RING TRACTION DEVICE**

(71) Applicant: Okumura Co., Ltd., Matsuzaka-shi Mie 515-0841 (JP); The Jikei University, Tokyo 105-8461 (JP)
(72) Inventor: SUMIYAMA Kazuki, Tokyo 105-8471 (JP); TANAKA Hiroto, Matsuzaka-shi, Mie 515-0841 (JP); MIYABATA Yusuke, Matsuzaka-shi, Mie 515-0841 (JP); KAMBA Shunsuke, Tokyo 105-8471 (JP)
(74) Representative: Vossius & Partner Patentanwälte Rechtsanwälte mbB
(86) International application number: PCT/JP2019/027070
(87) International publication number: WO 2021/005698

(57) **Abstract**

A detachable multi-ring traction device is inserted into a body through a forceps channel of an endoscope. When a lesion mucosa in the body is resected through an operation performed outside the body, the detachable multi-ring traction device is configured to be fixed by a clip to the lesion mucosa and a normal mucosa in order to pull the lesion mucosa, the normal mucosa opposing the lesion mucosa. The detachable multi-ring traction device includes toroidal rings each including a core curve and a meridional disk, the rings being coupled to each other such that outer edges of two adjacent ones of the rings are in contact with each other and the rings are connected to each other in a diametrical direction of the core curves.

## Description

### TECHNICAL FIELD

The present disclosure relates to a medical detachable multi-ring traction device.

### BACKGROUND ART

Recently, early-stage cancers that have occurred in a mucosa of a digestive tract can be efficiently resected through endoscopic submucosal dissection (ESD) in a minimally invasive manner. An operator who performs ESD operates surgery with the following procedure using, for example, an endoscope, a grasping tool, and a high-frequency knife.

First, the operator accurately determines the position of a lesion in a mucosa and incises the entire periphery of the mucosa surrounding the lesion. Next, the operator holds the entirely-incised mucosa with a clip. Then, the operator incises and dissects the submucosa while pulling a traction device coupled to the clip and lifting the mucosa.

In ESD, treatment needs to be performed in a limited view of the endoscope. Thus, during the incision of the submucosa and the dissection of the mucosa, the mucosa being incised may cover the visual field or treatment field of the endoscope. Such a state may obstruct the treatment, increase the risk of perforation, and lengthen the treatment time.

Patent Literature 1 discloses an example of technique that solves such a problem.

### CITATION LIST

### Patent Literature

Patent Literature 1: Japanese Laid-Open Patent Publication No. 2008-62004

### SUMMARY OF INVENTION

### Technical Problem

In the technique of Patent Literature 1, a mucosa cannot be incised in a sufficiently efficient manner. Thus, there is room for further improvement.

It is an object of the present disclosure to provide a traction device that allows for efficient incision and dissection of a mucosa.

### Solution to Problem

The summary described herein provides a simplified introduction to what is further described in the detailed description below. The summary is not intended to identify key features or essential features described in the claims, nor is it intended to be used as an aid in determining the scope of the claims.

A detachable multi-ring traction device according to an aspect of the present disclosure is inserted into a body through a forceps channel of an endoscope. When a lesion mucosa in the body is resected through an operation performed outside the body, the detachable multi-ring traction device is configured to be fixed by a clip to the lesion mucosa and a normal mucosa in order to pull the lesion mucosa, the normal mucosa opposing the lesion mucosa. The detachable multi-ring traction device includes toroidal rings each including a core curve (a large circle in plain view) and a meridional disk (a small circle as a cross-section), the rings being coupled to each other such that outer edges of two adjacent ones of the rings are in contact with each other and the rings are connected to each other in a diametrical direction of the core curves.

### BRIEF DESCRIPTION OF DRAWINGS

Fig. 1(A) is a front view of a detachable multi-ring traction device according to an embodiment.
Fig. 1(B) is a plan view of Fig. 1(A).
Fig. 1(C) is a right side view of Fig. 1(A).
Fig. 2 is a front view showing one of the rings of the detachable multi-ring traction device in Fig. 1(A).
Fig. 3 is a schematic diagram showing a first method for using the detachable multi-ring traction device in Fig. 1(A).
Fig. 4 is a schematic diagram showing a second method for using the detachable multi-ring traction device in Fig. 1(A).
Figs. 5(A) to (C) are pictures each showing a method for drawing the detachable multi-ring traction device in Fig. 1(A) into a body using grasping forceps.
Figs. 6(A) to (D) are pictures each showing a method for drawing the detachable multi-ring traction device in Fig. 1(A) into the body using a hemostatic clip.
Figs. 7(A) to (C) are pictures each showing a method for resecting the lesion mucosa using the detachable multi-ring traction device in Fig. 1(A).
Figs. 8(A) to (C) are pictures each showing a method for resecting the lesion mucosa using the detachable multi-ring traction device in Fig. 1(A).
Figs. 9(A) to (C) are pictures each showing a method for resecting the lesion mucosa using the detachable multi-ring traction device in Fig. 1(A).

### DESCRIPTION OF EMBODIMENTS

A detachable multi-ring traction device according to an embodiment will now be described with reference to the drawings. The technical scope of the present disclosure is not limited to these embodiments but may be modified in various forms without changing the gist of the invention.

### Structure of Detachable Multi-Ring Traction Device

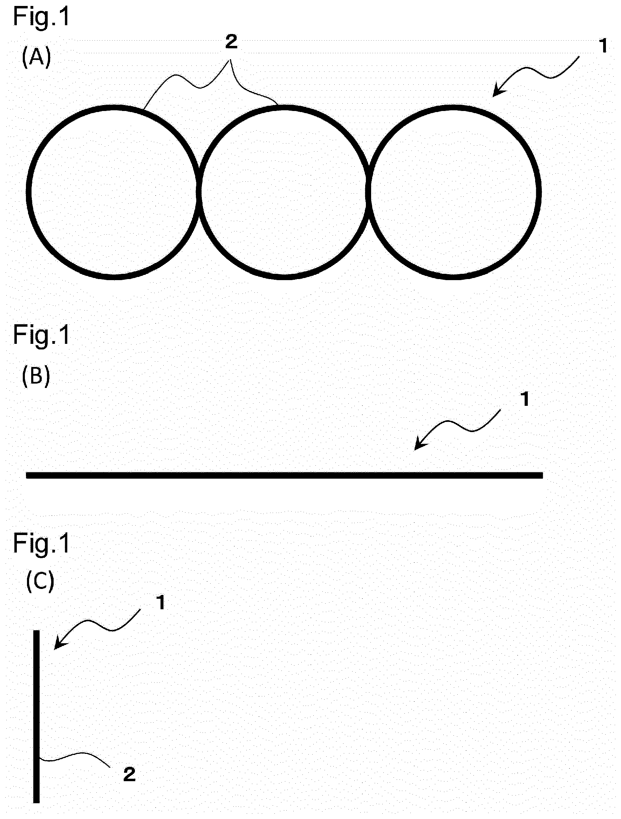

Figs. 1(A) to 1(C) each show a detachable multi-ring traction device 1 (hereinafter simply referred to as device 1) of the present embodiment. Fig. 1(A) is a front view of the device 1. Fig. 1(B) is a plan view of the device 1. Fig. 1(C) is a right side view of the device 1. The back view would be depicted in the same manner as the front view. The bottom view would be depicted in the same manner as the plan view. The left side view would be depicted in the same manner as the right side view.

The device 1 is configured to be inserted into a body through a forceps channel of a known endoscope (not shown). Further, when a lesion mucosa in the body is resected through an operation performed outside the body, the device 1 is configured to be fixed by a clip to the lesion mucosa and a normal mucosa in order to pull the lesion mucosa. The normal mucosa opposes the lesion mucosa.

The device includes multiple (e.g., three to five) toroidal rings 2 each including a core curve and a meridional disk. The rings 2 are coupled to each other such that the outer edges of two adjacent ones of the rings 2 are in contact with each other and these rings 2 are connected to each other in the diametrical direction of the core curves.

The rings 2 do not have to be circular. For example, the rings 2 may be slightly distorted or may be oval.

In an example in which the device 1 includes three rings 2, the ring 2 at one end is referred to as the first ring 2, the ring 2 at the other end is referred to as the third ring 2, and the ring 2 at the center is referred to as the second ring 2.

The rings 2 are made of the same material. The material of the rings 2 is preferably synthetic resin. The synthetic resin does not include rubber. The synthetic resin is preferably at least one selected from polyethylene, polypropylene, and polyvinyl chloride.

The device 1, that is, the rings 2 connected to each other in a row are integrally molded by injection-molding synthetic resin (e.g., polyethylene) into a mold. Alternatively, the device 1 may be formed as follows. That is, thread-like materials each having the diameter of the meridional disk of the ring 2 are used to form toroidal rings each having the diameter of the core curve. Then, ultrasonic welding is performed to connect a given number of the rings to each other.

As another option, the following method may be employed. That is, thread-like materials each having the diameter of the meridional disk of the ring 2 are used to form toroidal rings each having the diameter of the core curve. Then, the rings are arranged in a row in the diametrical direction of the core curve so that adjacent ones of the rings are welded using ultrasonics.

The device 1 preferably pulls lesion mucosae using the tensile strength of the mucosae, rather than actively pulling the lesion mucosae using the elasticity of rubber. Thus, the device 1 is made of plastic (synthetic resin), instead of elastic rubber. Instead of being elastic, the plastic is deformable so as to be insertable into the forceps channel of the endoscope together with a hemostatic clip or grasping forceps and is deformable in a direction in which the plastic is folded such that the core curves of the ring 2 overlap each other.

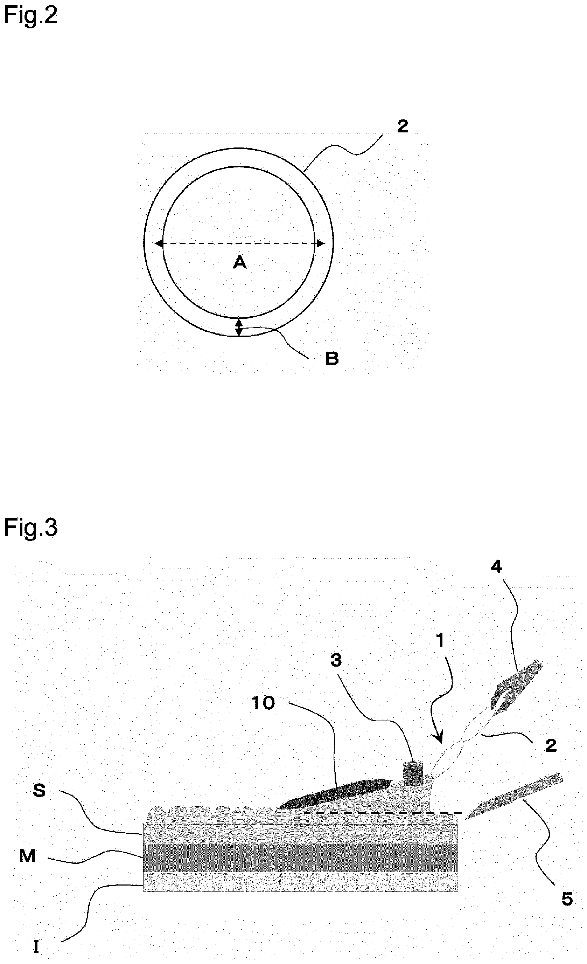

Fig. 2 is an enlarged view of one of the rings 2 of the device 1. In Fig. 2, the dimension is changed such that the meridional disk (the thickness of the ring 2) becomes more noticeable.

Diameter A of the core curve of each ring 2 is, for example, 5 mm to 10 mm and is preferably 6 mm to 8 mm. Diameter B of the meridional disk of each ring 2 is, for example, 0.1 mm to 0.5 mm and is preferably 0.2 mm to 0.4 mm.

The connected rings 2 are bendable and deformable such that the rings 2 can pass through the forceps channel. Further, the connected rings 2 are extendable and deformable in the diametrical direction of each core curve. The rings 2 do not need to have rubber-like elasticity.

### Method 1 for Using Detachable Multi-Ring Traction Device

Fig. 3 shows method 1 for using the device 1. The lower part of the figure shows a side cross-sectional view of the digestive tract (e.g., stomach or large intestine). The digestive tract includes a surface mucous layer S, a muscular layer M, and an inner serosa layer I that are arranged in order from the side of a lumen (the upper side in the figure). In this example, a lesion 10 remains in the surface mucous layer S.

An operator uses a knife 5 (e.g., high-frequency knife) to make a cut in the surface mucous layer S so as to surround a lesion mucosa including the lesion 10, which needs to be resected. The cut typically has a substantially circular shape or an oval shape with an appropriate size. Normally, the cut is set to be wider than the lesion 10 by a certain width.

Then, the operator uses a clip 3 to fix the first ring 2 of the device 1 on the inner side of the lesion mucosa including the lesion 10, that is, on the inner side of the cut. In this step, the operator uses forceps 4 to pull the second ring 2 or the third ring 2 with a certain force. The operator detaches the lesion mucosa using the knife 5 while pulling the lesion mucosa using the device 1.

### Method 2 for Using Detachable Multi-Ring Traction Device

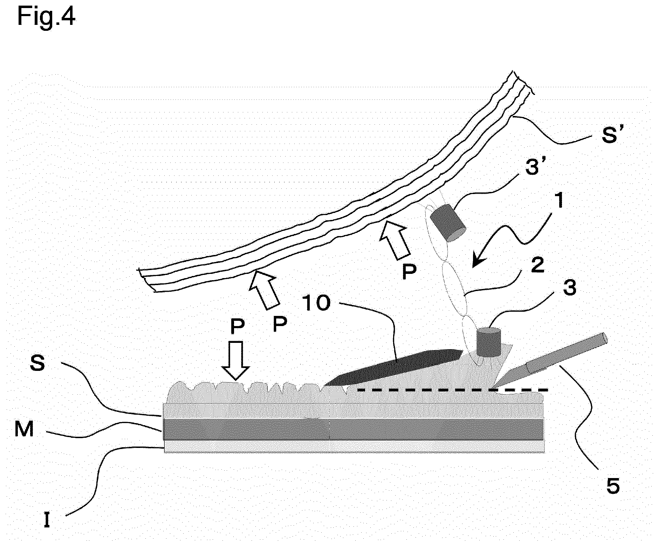

Fig. 4 shows method 2 for using the device 1. Like or the same reference numerals are given to those components that are like or the same as the corresponding components of Fig. 3, and detailed explanations are omitted.

In the usage method 2, the operator uses the knife 5 to make a cut having a certain size in the surface mucous layer S of the lesion mucosa including the lesion 10. Then, the operator uses the clip 3 to fix the first ring 2 of the device 1 to the lesion mucosa, which needs to be resected. Subsequently, the operator uses a clip 3' to fix the third ring 2 of the device 1 to a normal mucosa S' (a mucosa that is not to be resected). The normal mucosa S' opposes the lesion mucosa.

Next, the operator draws air into an organ (a stomach in this example) with a certain pressure and produces a given pressure P. This separates the clips 3, 3' from each other and pulls the device 1. Further, the device 1 pulls the lesion 10. In this state, the operator uses the high-frequency knife 5 to cut the lower layer of the lesion mucosa and dissect the lesion mucosa.

In addition to, or instead of, pulling the device 1 through the use of air pressure, the operator may use the high-frequency knife 5 to cut the lower layer of the lesion mucosa while pulling the device 1 fixed to the clips 3, 3'.

After detaching the lesion mucosa including the lesion 10, the operator cuts the first ring 2 of the device 1. For example, the operator may cut the first ring 2 by strongly pulling the first ring 2 with forceps or may cut the first ring 2 using the high-frequency knife 5. Subsequently, the operator collects the lesion mucosa including the lesion 10 and performs, for example, pathological examination.

The clip 3' left in the normal mucosa in the above-described operation naturally drops after a while and is then discharged out of the body.

### Method for Drawing Detachable Multi-Ring Traction Device into Body

A situation in which the device 1 is inserted into the body through the forceps channel of the endoscope will now be described.

### 1. Method Using Grip Forceps

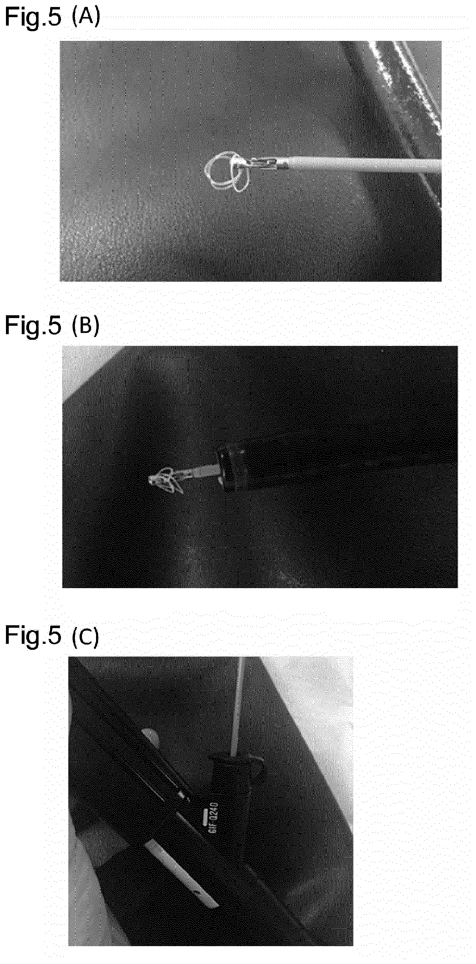

Figs. 5(A) to 5(C) show a procedure for attaching the device 1 to the grasping forceps. First, the operator folds the device 1 at the connection portions of the three rings 2 and then uses the grasping forceps to pinch all of the three rings 2 that have the same size as one ring 2 (refer to Fig. 5A). Using the grasping forceps, the operator moves the device 1 through the forceps channel (refer to Figs. 5(B) and 5(C)). Without folding the device 1, the operator may use the grasping forceps to pinch only one ring 2 (e.g., first ring 2) and draw the device 1 into the body through the forceps channel.

### 2. Method Using Hemostatic Clip

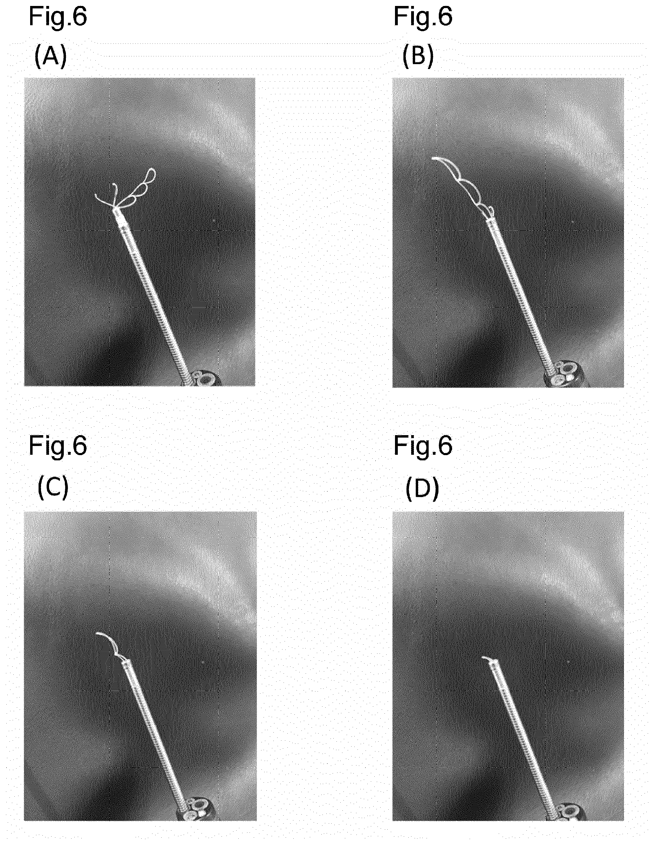

Figs. 6A to 6D show a situation in which the device 1 is pinched at the distal end of the hemostatic clip and drawn into the body. The operator hooks the first ring 2 of the device 1 on the basal end of the hemostatic clip that has been drawn from the distal end of a sheath (refer to Fig. 6A). Then, the operator draws the hemostatic clip into the sheath. This causes the device 1 to be inserted into the sheath (refer to Figs. 6(B), 6(C), and 6(D)). By inserting the sheath into the forceps channel, the operator draws the device 1 into the body of a patient.

### Method for Using Detachable Multi-Ring Traction Device in Actual ESD

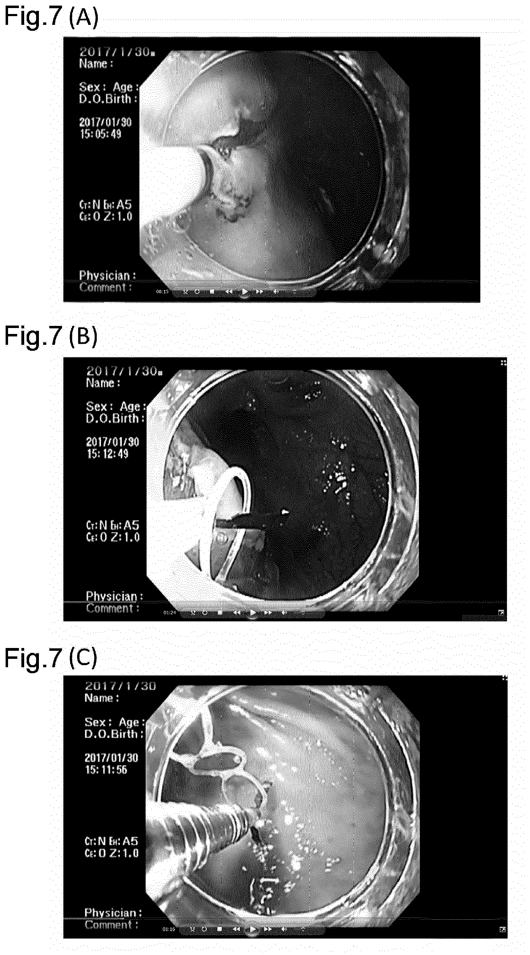

The procedure of ESD using the device 1 will now be described with reference to Figs. 7(A) to 9(C). This usage method is illustrated in Fig. 4.

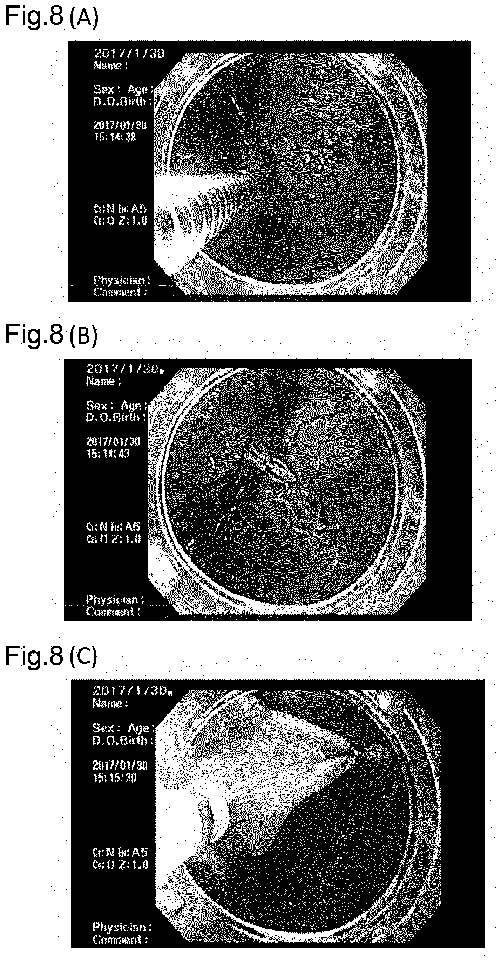

First, the operator uses the high-frequency knife to cut the surrounding mucosa (lesion mucosa), including a focus, in a substantially oval form (refer to Fig. 7(A)). Next, the operator hooks the first ring 2 of the device 1 on the clip 3 to fix the clip 3 to the lesion mucosa 10 (refer to Fig. 7(B)). Subsequently, the operator uses the clip 3' to fix the third ring 2 of the device 1 to the normal mucosa, which opposes the lesion mucosa. In this step, the device 1 connects between the clips 3, 3' with a certain tension (refer to Figs. 7(C) and 8(A)).

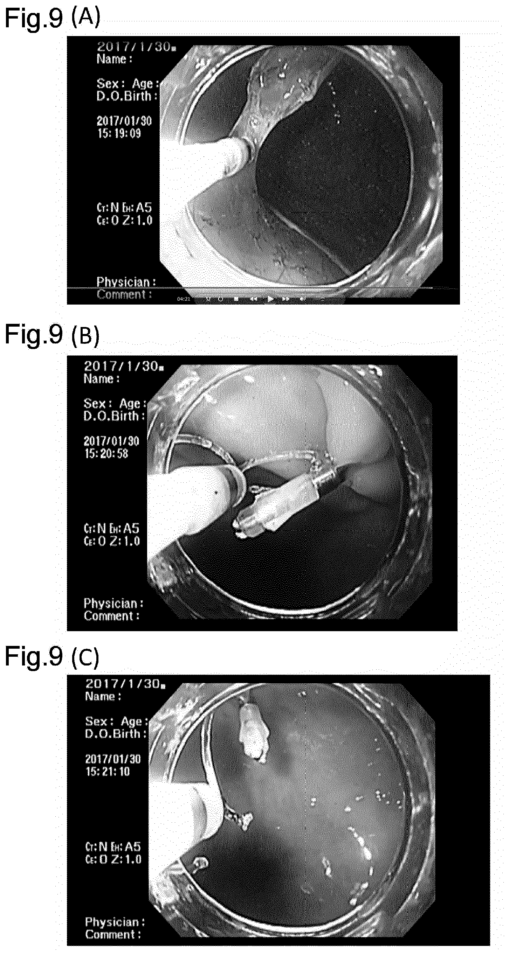

Then, the operator applies a certain inner pressure to the inside of the digestive tract to inflate the digestive tract. This causes the device 1 to pull the lesion mucosa 10 (refer to Fig. 8(B)). In this state, the operator uses the high-frequency knife to resect the exposed section of the lower layer of the exposed lesion mucosa 10 (refer to Fig. 8(C)). The operator resects the entire lower layer of the lesion mucosa 10 to detach the lesion mucosa 10 from the digestive tract (refer to Fig. 9(A)). Then, the operator uses the high-frequency knife to cut the third ring 2, which is fixed to the normal mucosa by the clip 3' (refer to Fig. 9(B)). This causes the device 1 and the lesion mucosa 10 to be detached from the digestive tract (refer to Fig. 9(C)). The clip 3'left in the digestive tract is naturally discharged after certain days.

In the present embodiment, during incision of the lower layer of a mucosa in ESD, the mucosa being incised does not cover the visual field or treatment field of the endoscope. This broadens the treatment field and allows for smooth and efficient incision of a lesion mucosa.

In the present embodiment, the device 1 includes three rings 2. The number of rings 2 may be changed. For example, the device 1 may include four or five rings 2. For example, a device 1 including three to five rings 2 is suitable for ESD performed for a stomach.

In the case of incision of a lesion mucosa 10 of a large intestine, even one or approximately two rings 2 may provide a sufficient length. Thus, when the device 1 is longer than a lesion mucosa, a ring at a certain position may be fixed to a clip without using the entire length of the rings. For example, the first ring 2 may be fixed to the lesion mucosa 10 using the clip 3 and the second ring 2 may be fixed to the normal mucosa using the clip 3'.

## Claims

1. A detachable multi-ring traction device inserted into a body through a forceps channel of an endoscope, wherein
when a lesion mucosa in the body is resected through an operation performed outside the body, the detachable multi-ring traction device is configured to be fixed by a clip to the lesion mucosa and a normal mucosa in order to pull the lesion mucosa, the normal mucosa opposing the lesion mucosa, and
the detachable multi-ring traction device comprises toroidal rings each including a core curve and a meridional disk, the rings being coupled to each other such that outer edges of two adjacent ones of the rings are in contact with each other and the rings are connected to each other in a diametrical direction of the core curves.

2. The detachable multi-ring traction device according to claim 1, wherein
the rings are made of a same material, and
the material is a synthetic resin that does not include rubber.

3. The detachable multi-ring traction device according to claim 2, wherein the synthetic resin is at least one selected from polyethylene, polypropylene, and polyvinyl chloride.
